# EUROPEAN PATENT SPECIFICATION

(11) **EP 3 707 238 B1**
(45) Date of publication and mention of the grant of the patent: **23.04.2025**
(21) Application number: 18800121.8
(22) Date of filing: 07.11.2018
(51) Int. Cl.: C12N 1/14, C12R 1/645, C12N 15/01, C02F 3/34, C12P 13/04, C12P 21/02, C12P 19/04, C02F 101/16

(54) **MICRO-ORGANISM TOLERANT FOR INORGANIC NITROGEN COMPOUNDS**
MIKROORGANISMUS, DER TOLERANT FÜR ANORGANISCHE STICKSTOFFVERBINDUNGEN IST
MICRO-ORGANISME TOLÉRANT POUR COMPOSÉS AZOTÉS INORGANIQUES

(30) Priority: 07.11.2017 EP 17200389
(43) Date of publication of application: 16.09.2020
(73) Proprietor: BioSolum B.V., 6003 DE Weert (NL)
(72) Inventor: SIEMERINK, Marco Antonius Josef, 6001 AC Weert (NL); KUIT, Wouter, 6001 AC Weert (NL)
(74) Representative: Wittop Koning, Tom Hugo
(86) International application number: PCT/EP2018/080523
(87) International publication number: WO 2019/092054

(56) References cited:
- CN-A- 102 465 105
- CN-A- 106 010 981
- CN-U- 203 112 574
- HOUBRAKEN J. ET AL: "Phylogeny of Penicillium and the segregation of Trichocomaceae into three families", STUDIES IN MYCOLOGY, vol. 70, 1 September 2011 (2011-09-01), NL, pages 1 - 51, XP055308503, ISSN: 0166-0616, DOI: 10.3114/sim.2011.70.01
- VAN DER HEYDEN C. ET AL: "Mitigating emissions from pig and poultry housing facilities through air scrubbers and biofilters: State-of-the-art and perspectives", BIOSYSTEMS ENGINEERING, vol. 134, 25 April 2015 (2015-04-25), pages 74 - 93, XP029230310, ISSN: 1537-5110, DOI: 10.1016/J.BIOSYSTEMSENG.2015.04.002

## Description

The invention relates to a novel nitrite tolerant organism, capable of using one or more inorganic nitrogen compounds as nitrogen source, while removing said one or more inorganic nitrogen compounds from a liquid source.

The term 'inorganic nitrogen compounds' as used herein, is intended to encompass at least ammonium, nitrite and/or nitrate or a combination of two or more thereof, unless otherwise indicated. Such inorganic nitrogen compounds are undesired compounds in waste/fertilizer streams, in particular in waste streams, originating from intensive livestock (of i.e. pigs, cattle, poultry), where high amounts of dung are produced. Said dung comprises high levels of inorganic nitrogen compounds, such as ammonium. Stables where such intensive livestock is placed, have a too high level of the odorous ammonia in the air, so that the air must be cleaned before being discharged to the environment. Such removal of ammonia usually takes place by so-called air scrubbers (e.g. biological and/or chemical), wherein a liquid is brought in contact with the polluted air and as a result, the ammonia dissolves in the liquid resulting in ammonium. In chemical air washers, the liquid is processed with sulphuric acid, which reacts with the ammonia to form an ammonium sulphate solution of low pH (about 1.5 - 4). Apart from the fact that such an acid end product may not be desired, the starting material sulphuric acid is a potential treat for the environment as well. In biological air washers, bacteria are present in the liquid and washer that are responsible for oxidation of ammonium into nitrite followed by further oxidation of the nitrite to nitrate. For this process, called nitrification, different species are known, e.g. *Nitrosomas* and *Nitrobacter.* The problem with the biological air scrubbers is that not all of the ammonium is converted to nitrate, resulting in the inorganic nitrogen compounds ammonium, nitrite and nitrate in the residual water. The residual waste water from biological air scrubbers usually has a pH of <5.0 to 6.5. For both biological and chemical air scrubbers, so-called combi-installations are known, wherein ammonia reduction is combined with odor and dust reduction. In biological combi air washers, the pH of the waste water can also be corrected to the neutral range, i.e. up to a pH of 7.5.

CN106010981 describes a nitrite degrading fungus, isolated from riverside sludge, capable of growing on standard Czapek's agar plates that contain 2.5 g/l sodium nitrite (~1.7 g/l nitrite). The pH of such plates is in the neutral range (i.e. 7.3 ± 0.2). In liquid standard Czapek's medium having a pH of 7.4, the said fungus is capable of nitrite removal at a nitrite concentration of 1 g/l.

CN203112574 describes a wastewater treatment device, wherein nitrite oxidizing Nitrobacter, derived from activated sludge, can be cultured at a nitrite content of 1 g/l N-NO₂, i.e. 3.3 g/l nitrite at a pH of 7.2 - 7.9. Nitrite is oxidized to nitrate, the most oxidized form of nitrogen.

CN102465105 describes a new Arthrobacter capable of denitrification, (i.e. the reduction of nitrite into molecular nitrogen) at a pH of 7.7 - 8.2, and has a nitrite tolerance of 800 mg/l.

Another example where elevated levels of inorganic nitrogen compounds are generated, is in breeding water of aquatic animal breeding, e.g. shrimps and fish. Such polluted breeding water is also often treated by addition of a cocktail of *Nitrosomas* and *Nitrobacter* species. WO2006/044499 describes a consortium of *Nitrosomas eutropha* and *Nitrobacter winogradskyi,* capable of nitrification at a pH of 8. Said consortium may work for small pollution as observed for shrimp breeding water.

The micro-organisms of the art are capable of nitrite removal at neutral or slightly basic pH. None of the known micro-organisms is suitable for removal of nitrite from aqueous liquids that are produced by the above described air scrubbers (possibly connected to reversed osmosis in order to concentrate the waste water for transport and further processing purposes; a 3 fold concentration can be obtained by reverse osmosis), due to the acidic pH and high concentrations of inorganic nitrogen compounds therein, where nitrite concentrations higher than 10 gram per liter nitrite are observed. Such acidic pH and high nitrite concentrations are toxic to the micro-organisms, presently known and used for removal of inorganic nitrogen compounds from liquid aqueous media.

Therefore, there is a need to provide a micro-organism that is tolerant for one or more inorganic nitrogen compounds at such high concentrations at acidic pH, that is also capable of removing said one or more inorganic nitrogen compounds, in particular from liquid waste such as biological or chemical air scrubber water, and preferably also of nitrification and/or nitrogen assimilation into organic nitrogen containing compounds.

The present inventors have now identified a hitherto unknown micro-organism deposited at the Westerdijk Fungal Biodiversity Institute (previously known as Centraalbureau voor Schimmelcultures (CBS-KNAW)) under accession number CBS143205, that is tolerant for at least nitrite, nitrate and/or ammonium and capable of nitrite, nitrate and ammonium removal (also at higher concentrations of nitrite, nitrate and/or ammonium in the medium), in particular at acidic pH, such as pH 3 - 6, more in particular at pH 4 - 6, 5 - 6 or 6.0. It was also found that the said micro-organism is capable of growing on and removal of nitrite, nitrate and ammonia at an acidic pH even below 3, and at neutral pH of up to 8 or even higher. Further, said micro-organism is capable of using ammonium, nitrite and nitrate as nitrogen source and assimilation of said inorganic nitrogen compounds into organic nitrogen containing compounds. The tolerance is preferably for nitrite, nitrate and/or ammonium, in particular for nitrite.

The micro-organism of the present disclosure preferably has an increased tolerance for inorganic nitrogen compounds, and has a nitrite tolerance of at least 200 mg/l, more preferably of at least 500 mg/l, even more preferably of 1 g/l nitrite, even more preferably of at least 2 g/l nitrite, even more preferably of at least 3.5 g/l nitrite, even more preferably of at least 5 g/l nitrite, even more preferably of at least 7 g/l nitrite, and most preferably of at least 10 g/l or even 12 g/l nitrite at a cultivation pH of 6.0. This means that the micro-organism can survive and utilize the inorganic nitrogen present (as nitrate, nitrite or ammonium) at such levels of nitrite at pH 6.0. The cultivation pH of the micro-organism of the present disclosure is preferably acidic, more preferably 2 - 6 or 3 - 6, still more preferably 4 - 6 and even more preferably 5 - 6. The indicated values for nitrite tolerance and nitrite clearance rate are therefore preferably valid for these pH ranges.

The micro-organism of the present disclosure preferably has a nitrite clearance rate of at least 100, more preferably at least 250, more preferably of at least 500 and most preferably of at least 630 mg/l/d, at a pH of 6.0. The starting concentration of nitrite is preferably 12.6 g/l. This means that the organism was capable to remove nitrite as nitrogen source in 250 ml shake flasks with 100 ml medium (biological air scrubber water supplemented with 200 mM phosphate and 30 g/l glucose; pH 6.0) at gentle agitation of 200 rpm and 20°C.

Further described is a method for the preparation of a monoculture of a micro-organism as described above, i.e. having a nitrite tolerance of at least 200 mg/l, more preferably of at least 500 mg/l, even more preferably of 1 g/l nitrite, even more preferably of at least 2 g/l nitrite, even more preferably of at least 3.5 g/l nitrite, even more preferably of at least 3.9 g/l nitrite, even more preferably of at least 5 g/l nitrite, even more preferably of at least 7 g/l nitrite, and most preferably of at least 10 g/l or even 12 g/l nitrite at a cultivation pH of 6.0, and a nitrite clearance rate at least 100, more preferably at least 250, more preferably of at least 500 and most preferably of at least 630 mg/l/d, at a pH of 6.0, comprising the steps of:
A) providing an aqueous medium having a pH of 2 - 6 and a nitrite concentration of at least 200mg/l,
B) supplementing the aqueous medium of step A) with an organic carbon source, to a carbon level corresponding with that of at least 1 g/l glucose and a phosphate source to a level of at least 1 mM,
C) allowing micro-organisms to grow,
D) inoculating micro-organisms from step C) into a fresh aqueous medium having a pH of 2 - 6 comprising a similar level of organic carbon source and phosphate as the aqueous medium after step B) and allow the micro-organisms to grow,
E) plating micro-organisms from step D) on a solid medium and allowing growth of single colonies thereon,
F) inoculating single colony micro-organisms from step E) into a fresh aqueous medium having a pH of 2 - 6 comprising a similar level of organic carbon source and phosphate as the aqueous medium after step B) and allow the micro-organisms to grow,
G) optionally repeat steps E) and F) until a monoculture of the micro-organism is obtained.

The aqueous medium in step A) can e.g. be residual water from a chemical or biological air scrubber as described above, or a mixture thereof, or any aqueous medium containing inorganic nitrogen compounds that need to be removed therefrom or need to be assimilated into organic nitrogen containing compounds. Used breeding water of aquatic animals or industrial residual water containing inorganic nitrogen compounds can also be used in step A). The nitrite concentration corresponds preferably with the envisaged nitrite tolerance of the micro-organism to be identified, and is preferably at least 500 mg/l, more preferably at least 1 g/l, at least 3.5 g/l, at least 3.9 g/l, even more preferably at least 5 g/l, more preferably at least 7 g/l, at least 10 g/l, and most preferably 12.6 g/l at a cultivation pH of 6.0. It is also possible to replace nitrite in step A) for another inorganic nitrogen compound such as ammonium or nitrate, in order to prepare a monoculture of a micro-organism that is tolerant for the said inorganic nitrogen compound. The pH of the aqueous medium is preferably 3 - 6, 4 - 6 and more preferably 5 - 6, even more preferably 5.5 - 6.0 or 6.0. The nitrite tolerance values as given above are preferably at pH 6.0, but can attractively be at the pH of the aqueous medium provided in step A.

In step B) the aqueous water is supplemented with a carbon and a phosphorous source. The carbon source can e.g. be glucose, whereas the phosphor source can e.g be a phosphate such as Na₂HPO₄ or KH₂PO₄. The amount of carbon is chosen such that it corresponds with the carbon provided by at least 1 g/l glucose, but preferably with 10 g/l glucose or even 30 g/l glucose. When growing on such medium, it has been observed that the amount of carbon needed seems to be depend on the amount of nitrogen, which needs to be removed.

The amount of phosphor is chosen such that it corresponds with at least 1 mM Na₂HPO₄, preferably at least 5 mM, more preferably at least 10 mM, more preferably at least 20 mM, more preferably at least 50 mM, more preferably at least 100 mM, most preferably at least 200mM Na₂HPO₄.

Subsequently, the micro-organisms are allowed to grow in step C), in particular for 3 weeks at 30°C and a pH typically around 6, preferably under mild agitation, such as at 200 rpm.

In step D), the micro-organisms of step C) are brought into an aqueous medium that has similar levels of carbon source and phosphate as the levels obtained in step B). For example, an inoculum of e.g.2 ml is taken from step C). Preferably, the aqueous medium in step D), is identical to that of step B). So if in step B) air scrubber residual water, supplemented with glucose and phosphate is used, the same medium is preferably used in step D). However, in step D), it is also possible to use a different aqueous medium, such as a growth medium comprising similar or preferably identical amounts of carbon source and phosphate. Preferably, the nitrite concentration and pH is similar to or the same of that of step A). Thereafter, the micro-organism is allowed to grow again.

Subsequently, the micro-organisms are allowed to grow on a solid growth medium in the form of single colonies. If a fungus is envisaged to be isolated, the medium is suitable for fungal growth, such as 2% PDA (Potato Dextrose Agar such as potato extract glucose broth manufactured by Carl Roth, Germany supplemented with 2% agar), comprising an antibiotic to prevent bacterial growth such as chloramphenicol at a concentration of e.g. 34 µg/ml. However, this method is also suitable to identify other micro-organisms than fungi, such as bacteria, and to this end, the growth medium can be made suitable for bacterial growth.

If necessary for obtaining a monoculture, the steps of inoculating and growing can be repeated until a significant fast growing micro-organism, in particular a fungus is able to grow on high concentration of inorganic nitrogen compounds in the medium, in particular nitrite. This way, the deposited micro-organism as described above has been found.

For growth of the micro-organism in steps B), D) and F), the organic carbon source is preferably supplemented to a carbon level corresponding with that of at least 10 g/l glucose. The phosphate source is preferably supplemented to a level of at least 200mM. The pH and nitrite content is preferably similar or the same as of the aqueous medium of step A).

Preferably, the carbon source comprises glucose, although also other carbon sources known to the skilled person can be used. It has surprisingly been found that the micro-organisms as described herein can use ethylene glycol and derivatives thereof as carbon source, even as sole carbon source. However, the growth is slightly better when grown on glucose. Therefore, the micro-organisms of the present disclosure can also be used for the removal of ethylene glycol and derivatives thereof, e.g. from antifreeze, coolant, hydraulic fluids, low-freezing dynamites and resins industry. High concentration of nitrite in the growth medium is not necessary, but still possible, for use of the micro-organism for removal of ethylene glycol from a liquid medium. It can be contemplated to combine air scrubber water from either biological or chemical air scrubbers or from both, with a liquid ethylene glycol containing waste medium, as the micro-organism of the present disclosure is capable of growth removal of both nitrite and ethylene glycol simultaneously.

By the above-described method, a micro-organism can be obtained having a nitrite tolerance of at least 200 mg/l nitrite, and/or a nitrite clearance rate of at least 100 mg/l/d nitrite, in particular of the preferred values as described above. Said tolerance and/or clearance rate are preferably at a pH of 2 - 6, or 3 - 6, more preferably 4 - 6, even more preferably 5 - 6 an most preferably at 6.0.

As will be further exemplified in the following examples, the micro-organism as described above preferably is a fungus belonging to the family of the *Trichocomaceae.*

Also disclosed is a method for clearing of inorganic nitrogen from a liquid source comprising inorganic nitrogen having an acid or neutral pH, i.e. of 2 - 8, comprising the steps of
a) contacting micro-organisms as described above with the liquid source,
b) allowing the micro-organisms to grow in the liquid source, providing the liquid source cleared from inorganic nitrogen.

By contacting the described micro-organisms with a liquid source comprising inorganic nitrogen, said inorganic nitrogen will be subjected to biological growth, resulting in clearing of nitrite, but also clearing of nitrate or ammonium present in the liquid. This was done by inoculation of the fungus into medium containing air scrubber water enriched with glucose and phosphate and grow for 20 days at 30°C and a pH of 6.0. It was found that the nitrite level of the liquid source could be brought from an initial level of 12,6 g/l nitrite to 0 g/l after 20 days at 30°C and a pH of 6.0, optionally at mild agitation, such as at 200 rpm. The pH can be in the range of 2 - 8, in particular 3 - 8, however, the pH is preferably acidic, such as between 3 and 6, preferably between 4 and 6 or more preferably between 5 and 6.

Also disclosed is a method for the preparation of one or more organic nitrogen containing compounds from a liquid source, comprising inorganic nitrogen, comprising the steps of
a) contacting micro-organisms as described above with the liquid source,
b) allowing the micro-organisms to grow in the liquid source,
c) purifying the one or more organic nitrogen containing compounds from the liquid source of step b).

The biomass of the fungi is grown as described above. The pH of the liquid medium is preferably between 3 and 8, more preferably between 4 and 7, even more preferably between 5 and 6, most preferably 5.5 - 6.0 or 6.0. The biomass of fungi comprises chitin. Due to the fact that the carbon source, such as glucose or ethylene glycol, and nitrogen are consumed while biomass is formed, it is most likely that the carbon and nitrogen are mainly used for biomass assimilation, such as (N-containing) carbohydrates, like chitin or derivatives thereof, proteins and lipids.

The one or more organic nitrogen containing compounds preferably are or comprise amino acids, such as in particular commercial interesting enzymes or proteins in general. Other nitrogen containing compounds, such as chitin or derivatives thereof can be prepared and purified as well. The term 'derivatives of chitin' is meant to encompass biomolecules, produced by the micro-organism wherein chitin is starting of intermediate material. The skilled person will be aware of such derivatives.

In the above methods, the liquid source is preferably derived from waste water from a biological or chemical air scrubber or a mixture thereof. Biological air scrubber waste water has a pH of usually <5 - 6.5 ( or up to 7.5 if neutralized), in particular around 6.5. Chemical air scrubber waste water has a more acidic pH of about 1.5 - 4. In order to bring the pH to the envisaged range of 3 - 6, in particular to 4 - 6, more in particular to 5 - 6 or 6.0, a sufficient amount of phosphate buffer can be added to both waste waters or to the mixture thereof, preferably in a concentration of 20 - 400 mM, more preferably 20 - 200 mM. If necessary, the pH can be further adjusted using a hydroxide, in particular sodium hydroxide, or an acid, such as hydrogen chloride. The skilled person will be familiar with the preparation of the suitable phosphate buffer by using KH₂PO₄ and/or K₂HPO₄ or a combination thereof and will know how to apply the hydroxide or acid to arrive at the envisaged pH.

The following examples are given to further illustrate the present disclosure.

### Example 1

### Preparation and identification of the novel micro-organism

The micro-organism of the present disclosure, i.e. as deposited as CBS143205, was identified in 250 ml shake flasks with 100 ml medium (biological air scrubber water supplemented with 200 mM phosphate and 10 g/l glucose; pH 6.0) at 200 rpm and 20°C. The micro-organisms were allowed to grow for 3 weeks at 20°C and a pH typically around 6. After 3 weeks, an inoculum of 2 ml was taken and brought into refreshed identical medium, based on residual air scrubber water, supplemented with glucose and phosphate, where after the micro-organism was allowed to grow again. These steps of inoculating and growing were repeated until a significant fast growing fungi was able to grow on high concentration of nitrite in the medium.

The nuclear rRNA cistron has been used for fungal diagnostics and phylogenetics for more than 20 year (2016 Begerow). Preliminary Next-Generation sequencing reveal at least 18 rRNAs and 70 tRNAs in the novel micro-organism, which are provided in SEQ ID No 1 - 88. The rRNAs and tRNAs sequences were identified with Barrnap and Aragorn. Barrnap is a software program that predicts the location of ribosomal RNA genes in genomes. It supports bacteria (5S,23S,16S), archaea (5S,5.8S,23S,16S), mitochondria (12S,16S) and eukaryotes (5S,5.8S,28S,18S). ARAGORN is a program commonly used to detect tRNA genes in nucleotide sequences ( http://mbio-serv2.mbioekol.lu.se/ARAGORN/).
The eukaryotic rRNA cistron consists of the 18S, 5.8S, and 28S rRNA genes transcribed as a unit by RNA polymerase I. Posttranscriptional processes split the cistron, removing two internal transcribed spacers. These two spacers, including the 5.8S gene, are usually referred to as the ITS region. The 18S nuclear ribosomal small subunit rRNA gene (SSU) and the 28S nuclear ribosomal large subunit rRNA gene (LSU) sometimes discriminates species on its own or combined with ITS. LSU, a favoured phylogenetic marker among many mycologists, had virtually no amplification, sequencing, alignment, or editing problems, and the barcode gap was superior to the SSU. However, across the fungal kingdom, ITS was generally superior to LSU in species discrimination and had a more clearly defined barcode gap (2016 Begerow). The ITS RNA sequence (643 nt) of this unidentified fungi was determined by PCR and has been designated SEQ ID No 89. Internet nucleotide blast search (https://blast.ncbi.nim.nih.gov/Blast.cgi?PROGRAM=blastn&PAGE TYPE=BlastSear ch&LINK LOC=blasthome; Database Nucleotide collection (nr/nt) using Megablast (Optimize for highly similar sequences) reveals an identity of only 91% with *Phialosimplex chlamydosporus,* suggesting a new genus and species.

Next to ITS, LSU and SSU, protein-coding genes are also widely used in mycology for phylogenetic analyses or species identification. For Ascomycota (including mold genera such as Aspergillus), they are generally superior to rRNA genes for resolving relationships at various taxonomic levels. Among protein-coding genes, the largest subunit of RNA polymerase II (RPB1) may have potential as a fungal barcode; together with two additional optional genes, namely the second largest subunit of RNA polymerase II (RPB2) and a gene encoding a minichromosome maintenance protein (MCM7).

The LSU, SSU, RPB1, RPB2, MCM7 were extracted from Next-Generation sequencing data and were designated SEQ ID No 90, 91, 92, 93, 94, respectively. LSU was found with the primers LR0R 5'-ACCCGCTGAACTTAAGC-3'; forward and LR5 5'-TCCTGAGGGAAACTTCG-3'; reverse and for SSU with the primers NS1 5'-GTAGTCATATGCTTGTCTC-3'; forward and NS4 5'-CTTCCGTCAATTCCTTTAAG-3'; reverse (http://www.fungalbarcoding.org/DefaultInfo.aspx?Page=Primers). The protein encoding sequences RPB1, RPB2, MCM7 were found in the annotated preliminary next generation sequence data.

Internet nucleotide blast search (https://blast.ncbi.nlm.nih.gov/Blast.cgi?PROGRAM=blastn&PAGE TYPE=BlastSear ch&LINK LOC=blasthome; Database Nucleotide collection (nr/nt) using Megablast (Optimize for highly similar sequences) of SEQ ID No 90, 91, 92, 93, 94 revealed an identity of only 97%, 94%, 78%(1^{st} part)/75% (2^{nd} part), 80%, 78% with *Sagenomella sp. HMH-2007a* 28S ribosomal RNA gene, partial sequence; *Trichocoma paradoxa* gene for 18S rRNA, partial sequence, strain:IFO 6765; *Aspergillus niger* contig An01c0390, genomic contig/*Aspergillus terreus NIH2624* conserved hypothetical protein (ATEG_00681) partial mRNA; *Aspergillus niger* contig An12c0030, genomic contig; *Aspergillus fischeri* NRRL 181 DNA replication licensing factor Mcm7, putative partial mRNA, respectively.

Therefore, the present disclosure also encompasses a micro-organism having an ITS RNA sequence having at least 97%, preferably at least 98, or 99% sequence identity with SEQ ID NO 89. The tRNA and rRNA of said micro-organism preferably has a sequence identity with SEQ ID Nos 1 - 88 (with a query coverage of 100%) of at least 95%, more preferably of at least 98%, or 99%. Said micro-organism preferably has a sequence identity of at least 97%, preferably at least 98% or more preferably of at least 99% with SEQ ID NO 90-94.

### Example 2

Clearing of inorganic nitrogen from biological air scrubber waste water The micro-organism of the present disclosure, i.e. as deposited as CBS143205, was inoculated in 250 ml shake flasks with 100 ml biological air scrubber water supplemented with 200 mM phosphate and 10 g/l glucose; pH 6,0) at 200 rpm and 20°C. The micro-organisms were allowed to grow for 19 days at 30°C and a pH of 6.

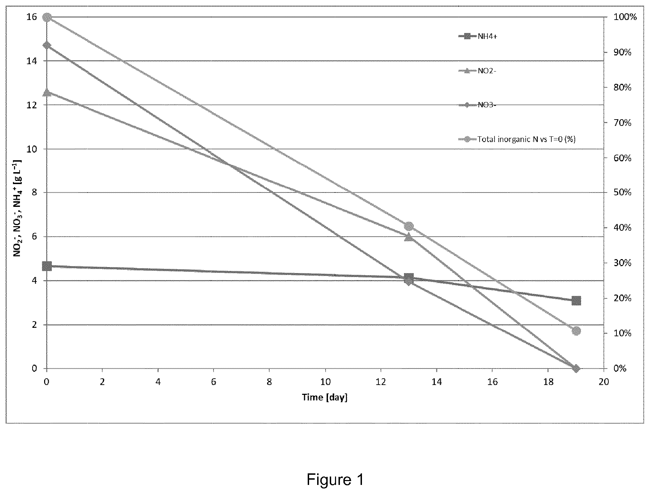

The concentrations of the inorganic nitrogen compounds of the biological air scrubber water are depicted in table 1 and figure 1, wherein concentration of nitrite, nitrate, and ammonium of biological air scrubber water are depicted in time.

**Table 1: Concentration of inorganic compounds in g/l in (concentrated) biological air scrubber water in time**

| | 0 days | 13 days | 19 days |
|---|---|---|---|
| NH₄⁺ | 4.7 | 4.1 | 3.1 |
| NO₂⁻ | 12.6 | 6.0 | 0.0 |
| NO₃⁻ | 65.1 | 17.5 | 0.0 |

### Example 3

Clearing of inorganic nitrogen from chemical air scrubber waste water The micro-organism of the present disclosure was inoculated in 100 mL shake flasks with 50 mL medium (chemical air scrubber water; brought to a pH of 6.0 by supplementation with 100 mM phosphate; 30 g/L glucose) at 200 rpm and 20°C. Spore elements were supplemented to a final concentration of 1,2 mM H₃BO₃, 40 µM CuSO₄, 3 µM Co(NO₃)₂, 227 µM MnCl₂, 41 µM Na₂MoO₄, 94 µM ZnSO₄, 14 mM MgSO₄, 18 mM CaCl₂, 1 mM C₆H₈O₇, 290 µM (NH₄)₅Fe(C₆H₄O₇)2, 67 µM Na₂EDTA. The micro-organisms were allowed to grow for 19 days at 30°C and a pH of 6.

The concentrations of the inorganic nitrogen compounds of the chemical air scrubber water are depicted in table 2, wherein concentration of nitrite, nitrate, and ammonium of chemical air scrubber water are depicted in time.

**Table 2: Concentration of inorganic compounds in g/l in (concentrated) biological air scrubber water in time**

| | 0 days | 13 days | 19 days |
|---|---|---|---|
| NH₄⁺ | 38.0 | 30.0 | 20.0 |
| NO₂⁻ | 0.0 | 0.0 | 0.0 |
| NO₃⁻ | 0.0 | 0.0 | 0.0 |

### Example 4

### Clearing of inorganic nitrogen from a mixture of biological and chemical air scrubber waste water

The micro-organism of the present disclosure was inoculated in 250 mL shake flasks with 40 ml chemical air scrubber water (set at a pH of 6.0) and 50 ml biological air scrubber water, 200 mM phosphate (pH 6.0) and 30 g/l glucose at 200 rpm and 20°C. The micro-organisms were allowed to grow for 19 days at 30°C and a pH of 6.

The concentrations of the inorganic nitrogen compounds of the chemical air scrubber water are depicted in table 3, wherein concentration of nitrite, nitrate, and ammonium of chemical air scrubber water are depicted in time.

**Table 3: Concentration of inorganic compounds in g/l in (concentrated) mixed air scrubber water in time**

| | 0 days | 13 days | 19 days |
|---|---|---|---|
| NH₄⁺ | 24.3 | 19.5 | 13.7 |
| NO₂⁻ | 3.6 | 3.2 | 0.0 |
| NO₃⁻ | 6.7 | 2.8 | 0.0 |

It was observed that any mixture between waste water of biological air scrubbers and chemical air scrubbers could be used as material for nitrite removal and nitrogen assimilation, as long as the pH was brought in the range 3 - 8, preferably to 4 - 6, more preferably in particular in the range 5 - 6. This can be done by mixing chemical with biological air scrubber waste waters in any ratio, the neutralized chemical waste water having a pH as low as 1.5 - 4 to a pH of 6, the biological waste water having a pH of 5 - 7.5, as long as the final pH is within the range of 3 - 8, preferably 4 - 6, more preferably 5 - 6, even more preferably 5.5 - 6.0. To this end, the neutralized chemical air scrubber waste water is preferably brought to the envisaged pH and subsequently mixed with the biological air scrubber waste water, as mixing the high nitrite containing biological air scrubber water with the acidic chemical air scrubber water may cause undesired formation of nitrous and nitric oxide. To bring the waste waters or the mixture to the envisaged pH, phosphate buffer can be used, e.g. by the addition of KH₂PO₄ and/or K₂HPO₄, and, if still necessary, addition of the required amount of sodium hydroxide or hydrogen chloride is possible.

### Example 5

### Clearing of inorganic nitrogen from biological air scrubber waste water with ethylene glycol as sole carbon source

The micro-organism of the present disclosure was inoculated in 250 mL shake flasks with 100 mL biological air scrubber water supplemented with 200 mM phosphate and 10 g/l ethylene glycol; pH 6,0) at 200 rpm and 20°C. The micro-organisms were allowed to grow for 19 days at 30°C and a pH of 6.

The concentrations of the inorganic nitrogen compounds of the biological air scrubber water are depicted in table 4, wherein concentration of nitrite, nitrate, and ammonium of biological air scrubber water are depicted in time.

**Table 4: Concentration of inorganic compounds in g/l in (concentrated) biological air scrubber water in time, ethylene glycol as sole carbon source.**

| | 0 days | 13 days | 19 days |
|---|---|---|---|
| NH₄⁺ | 4.7 | 3.8 | 2.9 |
| NO₂⁻ | 12.6 | 5.7 | 0.0 |
| NO₃⁻ | 65.1 | 16.6 | 0.0 |

It was observed that the micro-organism had a somewhat slower growth as compared to growth on a medium having a similar glucose content instead of ethylene glycol. Similar results were observed for ethylene glycol levels up to 30 g/l.

### Example 6

### Growth on and clearance of ethylene glycol

The micro-organism of the present disclosure was inoculated in 250 ml shake flasks with 100 ml aqueous growth medium, i.e. water supplemented with 30 g/l ethylene glycol, 100mM phosphate pH 6.0, 25.9 g/l ammonium chloride, 1,2 mM H₃BO₃, 40 µM CuSO₄, 3 µM Co(NO₃)₂, 227 µM MnCl₂, 41 µM Na₂MoO₄, 94 µM ZnSO₄, 14 mM MgSO₄, 18 mM CaCl₂, 1 mM C₆H₈O₇, 290 µM (NH₄)₅Fe(C₆H₄O₇)2, 67 µM Na₂EDTA), at 200 rpm and 20°C. The micro-organisms were allowed to grow for 19 days at 30°C and a pH of 6.

The concentrations of ammonium and ethylene glycol of the defined media are depicted in table 5, wherein concentration is depicted in time.

**Table 5: Concentration of inorganic compounds in g/l in media in time, ethylene glycol as sole carbon source, NH₄⁺ as sole nitrogen source.**

| | 0 days | 13 days | 19 days |
|---|---|---|---|
| NH₄⁺ | 8.7 | 5.2 | 4.1 |
| Ethylene glycol | 30 | 20 | 8 |

## Claims

1. Micro-organism, deposited at the Westerdijk Fungal Biodiversity Institute under accession number CBS143205.

2. Method for the preparation of a monoculture of a micro-organism having a nitrite tolerance of at least 200 mg/l nitrite and a nitrite clearance rate of at least 100 mg/l/d nitrite at a pH of 6.0, comprising the steps of:
A) providing an aqueous medium having a pH of 2 - 6.0 and a nitrite concentration of at least 200 mg/l,
B) supplementing the aqueous medium of step A) with an organic carbon source, to a carbon level corresponding with that of at least 1 g/l glucose and a phosphate source to a level of at least 20 mM,
C) allowing micro-organisms to grow,
D) inoculating micro-organisms from step C) into a fresh aqueous medium comprising the same level of organic carbon source, phosphate and nitrite as the aqueous medium after step B) and allow the micro-organisms to grow,
E) plating micro-organisms from step D) on a solid medium and allowing growth of single colonies thereon,
F) inoculating single colony micro-organisms from step E) into a fresh aqueous medium comprising the same level of organic carbon source, phosphate and nitrite as the aqueous medium after step B) and allow the micro-organisms to grow,
G) optionally repeat steps E) and F) until a monoculture of the micro-organism is obtained.

3. Method of claim 2, wherein the pH of the aqueous liquid is 3 - 6.0, preferably 4 - 6.0, more preferably 5 - 6.0, even more preferably 6.0.

4. Method of claim 2 or 3, wherein in step B) the organic carbon source is supplemented to a carbon level corresponding with that of at least 10 g/l glucose and/or wherein the phosphate source is supplemented to a level of at least 200mM.

5. Method of any of claims 2 - 4, wherein the carbon source comprises glucose or ethylene glycol.

6. Method of any of the claims 2 - 5, wherein the micro-organism has a nitrite tolerance of at least 500 mg/l, preferably of 1 g/l nitrite, more preferably of at least 2 g/l nitrite, even more preferably of at least 3.5 g/l nitrite, even more preferably of at least 5 g/l nitrite, even more preferably of at least 7 g/l nitrite, even more preferably of at least 10 g/l and most preferably of at least 12 g/l nitrite at a cultivation pH of 6.0.

7. Method of any of the claims 2 - 6, wherein the micro-organism has a nitrite clearance rate of at least 100 mg/l/d nitrite, preferably of at least 250, more preferably of at least 500 and most preferably of at least 630 mg/l/d, at a pH of 6.0.

8. Method of any of the claims 2 - 7, wherein the micro-organism is a fungus belonging to the family of the *Trichocomaceae.*

9. Method for clearing of inorganic nitrogen from a liquid source having a pH of 2 - 8 comprising inorganic nitrogen comprising the steps of
a) contacting a micro-organism of claim 1 with the liquid source,
b) allowing the micro-organisms to grow in the liquid source, providing the liquid source cleared from inorganic nitrogen.

10. Method for the preparation of one or more organic nitrogen containing compounds from a liquid source comprising inorganic nitrogen, comprising the steps of
a) contacting micro-organism of claim 1 with the liquid source,
b) allowing the micro-organisms to grow in the liquid source,
c) purifying the one or more organic nitrogen containing compounds from the liquid source of step b).

11. Method according to claim 9 or 10, wherein the pH of the liquid source is 2 - 8, preferably 4 - 6, more preferably 5 - 6, even more preferably 6.0.

12. Method according to any of the claims 9 - 11 wherein the liquid source is derived from wastewater from a biological or chemical air scrubber or a mixture thereof.

## Patentansprüche

1. Mikroorganismus, hinterlegt beim Westerdijk Fungal Biodiversity Institute unter der Hinterlegungsnummer CBS143205.

2. Verfahren zur Herstellung einer Monokultur eines Mikroorganismus mit einer Nitrit-Toleranz von mindestens 200 mg/l Nitrit und einer Nitrit-Clearance-Rate von mindestens 100 mg/I/Tag Nitrit bei einem pH-Wert von 6,0, umfassend die folgenden Schritte:
A) Bereitstellen eines wässrigen Mediums mit einem pH-Wert von 2 - 6,0 und einer Nitritkonzentration von mindestens 200 mg/l,
B) Ergänzen des wässrigen Mediums aus Schritt A) mit einer organischen Kohlenstoffquelle bis zu einem Kohlenstoffgehalt, der dem von mindestens 1 g/l Glucose entspricht, und einer Phosphatquelle bis zu einem Gehalt von mindestens 20 mM,
C) Wachsenlassen von Mikroorganismen,
D) Beimpfen von Mikroorganismen aus Schritt C) in ein frisches wässriges Medium, das den gleichen Gehalt an organischer Kohlenstoffquelle, Phosphat und Nitrit wie das wässrige Medium nach Schritt B) enthält, und Wachsenlassen der Mikroorganismen,
E) Ausplattieren von Mikroorganismen aus Schritt D) auf ein festes Medium und Wachsenlassen von Einzelkolonien darauf,
F) Beimpfen einzelner Kolonien von Mikroorganismen aus Schritt E) in ein frisches wässriges Medium, das den gleichen Gehalt an organischer Kohlenstoffquelle, Phosphat und Nitrit wie das wässrige Medium nach Schritt B) enthält, und Wachsenlassen der Mikroorganismen,
G) gegebenenfalls Wiederholung der Schritte E) und F), bis eine Monokultur des Mikroorganismus erhalten wird.

3. Verfahren nach Anspruch 2, wobei der pH-Wert der wässrigen Flüssigkeit 3 - 6,0, vorzugsweise 4 - 6,0, weiter bevorzugt 5 - 6,0, noch weiter bevorzugt 6,0 beträgt.

4. Verfahren nach Anspruch 2 oder 3, wobei in Schritt B) die organische Kohlenstoffquelle auf einen Kohlenstoffgehalt ergänzt wird, der dem von mindestens 10 g/l Glucose entspricht, und/oder wobei die Phosphatquelle auf einen Gehalt von mindestens 200 mM ergänzt wird.

5. Verfahren nach einem der Ansprüche 2 bis 4, wobei die Kohlenstoffquelle Glukose oder Ethylenglykol umfasst.

6. Verfahren nach einem der Ansprüche 2 bis 5, wobei der Mikroorganismus eine Nitrit-Toleranz von mindestens 500 mg/l, vorzugsweise von 1 g/l Nitrit, noch bevorzugter von mindestens 2 g/l Nitrit, noch bevorzugter von mindestens 3,5 g/l Nitrit, noch bevorzugter von mindestens 5 g/l Nitrit, noch bevorzugter von mindestens 7 g/l Nitrit, noch bevorzugter von mindestens 10 g/l und am meisten bevorzugt von mindestens 12 g/l Nitrit bei einem Kultivierungs-pH von 6,0 aufweist.

7. Verfahren nach einem der Ansprüche 2 bis 6, wobei der Mikroorganismus bei einem pH-Wert von 6,0 eine Nitrit-Clearance-Rate von mindestens 100 mg/I/Tag Nitrit, vorzugsweise von mindestens 250, noch bevorzugter von mindestens 500 und am meisten bevorzugt von mindestens 630 mg/I/Tag aufweist.

8. Verfahren nach einem der Ansprüche 2 bis 7, wobei der Mikroorganismus ein Pilz aus der Familie der *Trichocomaceae* ist.

9. Verfahren zur Reinigung von anorganischem Stickstoff aus einer flüssigen Quelle mit einem pH-Wert von 2 - 8, die anorganischen Stickstoff enthält, umfassend die Schritte
a) Inkontaktbringen eines Mikroorganismus nach Anspruch 1 mit der flüssigen Quelle,
b) Wachsenlassen der Mikroorganismen in der flüssigen Quelle, wodurch die von anorganischem Stickstoff gereinigte flüssige Quelle bereitgestellt wird.

10. Verfahren zur Herstellung einer oder mehrerer organischer stickstoffhaltiger Verbindungen aus einer flüssigen Quelle, die anorganischen Stickstoff enthält, umfassend die Schritte
a) Inkontaktbringen von Mikroorganismen nach Anspruch 1 mit der flüssigen Quelle,
b) Wachsenlassen der Mikroorganismen in der flüssigen Quelle,
c) Reinigen der einen oder mehreren organischen stickstoffhaltigen Verbindungen aus der flüssigen Quelle von Schritt b).

11. Verfahren nach Anspruch 9 oder 10, wobei der pH-Wert der flüssigen Quelle 2 - 8, vorzugsweise 4 - 6, noch bevorzugter 5 - 6, noch weiter bevorzugt 6,0 beträgt.

12. Verfahren nach einem der Ansprüche 9 bis 11, wobei die flüssige Quelle von Abwasser aus einem biologischen oder chemischen Luftwäscher oder einer Mischung davon stammt.

## Revendications

1. Micro-organisme, déposé au Westerdijk Fungal Biodiversity Institute sous le numéro d'accession CBS143205.

2. Méthode de préparation d'une monoculture d'un micro-organisme ayant une tolérance au nitrite d'au moins 200 mg/l de nitrite et un taux d'élimination du nitrite d'au moins 100 mg/l/jour de nitrite à un pH de 6,0, comprenant les étapes suivantes :
A) fournir un milieu aqueux ayant un pH de 2 à 6,0 et une concentration en nitrites d'au moins 200 mg/l,
B) compléter le milieu aqueux de l'étape A) avec une source de carbone organique, à un niveau de carbone correspondant à celui d'au moins 1 g/l de glucose et une source de phosphate à un niveau d'au moins 20 mM,
C) laisser les micro-organismes se développer,
D) inoculer les micro-organismes de l'étape C) dans un milieu aqueux frais comprenant la même teneur en source de carbone organique, en phosphate et en nitrite que le milieu aqueux après l'étape B) et laisser les micro-organismes se développer,
E) ensemencer les micro-organismes de l'étape D) sur un milieu solide et permettre la croissance de colonies uniques sur ce milieu,
F) inoculer les micro-organismes à colonie unique de l'étape E) dans un milieu aqueux frais comprenant le même niveau de source de carbone organique, de phosphate et de nitrite que le milieu aqueux après l'étape B) et laisser les micro-organismes se développer,
G) répéter éventuellement les étapes E) et F) jusqu'à l'obtention d'une monoculture du micro-organisme.

3. Méthode de la revendication 2, dans laquelle le pH du liquide aqueux est compris entre 3 et 6,0, de préférence entre 4 et 6,0, de préférence entre 5 et 6,0, et de préférence encore entre 6,0.

4. Méthode de la revendication 2 ou 3, dans laquelle, à l'étape B), la source de carbone organique est complétée à un niveau de carbone correspondant à celui d'au moins 10 g/l de glucose et/ou dans laquelle la source de phosphate est complétée à un niveau d'au moins 200 mM.

5. Méthode de l'une des revendications 2 à 4, dans laquelle la source de carbone comprend du glucose ou de l'éthylène glycol.

6. Méthode de l'une des revendications 2 à 5, dans laquelle le micro-organisme a une tolérance au nitrite d'au moins 500 mg/l, de préférence de 1 g/l de nitrite, de préférence d'au moins 2 g/l de nitrite, de préférence encore d'au moins 3,5 g/l de nitrite, de préférence encore d'au moins 5 g/l de nitrite, de préférence encore d'au moins 7 g/l de nitrite, de préférence encore d'au moins 10 g/l et de préférence encore d'au moins 12 g/l de nitrite, à un pH de culture de 6,0.

7. Méthode de l'une des revendications 2 à 6, dans laquelle le micro-organisme a un taux de clairance des nitrites d'au moins 100 mg/l/jour de nitrites, de préférence d'au moins 250, de préférence d'au moins 500 et de préférence d'au moins 630 mg/l/jour, à un pH de 6,0.

8. Méthode de l'une des revendications 2 à 7, dans laquelle le micro-organisme est un champignon appartenant à la famille des *Trichocomaceae.*

9. Méthode d'élimination de l'azote inorganique d'une source liquide ayant un pH de 2 à 8 et comprenant de l'azote inorganique, comprenant les étapes suivantes
a) mettre en contact un micro-organisme de la revendication 1 avec la source liquide,
b) laisser les micro-organismes se développer dans la source liquide, ce qui permet d'obtenir une source liquide débarrassée de l'azote inorganique.

10. Méthode de préparation d'un ou plusieurs composés organiques contenant de l'azote à partir d'une source liquide comprenant de l'azote inorganique, comprenant les étapes suivantes
a) mettre en contact le micro-organisme de la revendication 1 avec la source liquide,
b) laisser les micro-organismes se développer dans la source liquide,
c) purifier le ou les composés organiques contenant de l'azote à partir de la source liquide de l'étape b).

11. Méthode selon la revendication 9 ou 10, dans laquelle le pH de la source liquide est compris entre 2 et 8, de préférence entre 4 et 6, de préférence entre 5 et 6, de préférence encore à 6,0.

12. Méthode selon l'une des revendications 9 à 11, dans laquelle la source liquide est dérivée des eaux usées d'un épurateur d'air biologique ou chimique ou d'un mélange de ceux-ci.
